Europäisches Patentamt

European Patent Office

Office européen des brevets

(19)

(11) Publication number: **0 178 267**
A2

(12) **EUROPEAN PATENT APPLICATION**

(21) Application number: 85830247.4

(22) Date of filing: 02.10.85

(51) Int. Cl.⁴: **A 61 K 31/70**

---

(30) Priority: 09.10.84 IT 4898384

(43) Date of publication of application: 16.04.86
Bulletin 86/16

(84) Designated Contracting States: DE FR GB

(71) Applicant: Polifarma S.p.A., Via Tor Sapienza 138,
I-00155 Roma (IT)

(72) Inventor: Materazzi, Mario, 10 Via Delleani,
I-00155 Roma (IT)
Inventor: De Luca, Giovanna, 124 Via Ugo De Carolis,
I-00136 Roma (IT)
Inventor: Politi, Vincenzo, 77 Via Albano, I-00179 Roma
(IT)
Inventor: Di Stazio, Giovanni, 221 Via Clivo di Cinna,
I-00136 Roma (IT)

(74) Representative: Bazzichelli, Alfredo et al, c/o Società
Italiana Brevetti Piazza Poli 42, I-00187 Roma (IT)

---

(54) A uridine-based pharmaceutically active agent for the treatment of cerebral pathologies.

(57) A pharmaceutically active agent comprising uridine is
described. It may be used to block decay in neuron functional
activity due to cerebral aging and in any case in which neu-
rons are deprived of their normal level of energy materials
(hypoxemia, ischemia, hypoglycemia).

EP 0 178 267 A2

# A URIDINE-BASED PHARMACEUTICALLY ACTIVE AGENT FOR THE TREATMENT OF CEREBRAL PATHOLOGIES

## Background of the Invention

### 1. Field of the Invention

The present invention concerns uridine, a pharmaceutically active agent for cerebral pathologies in elderly patients, under conditions of cerebral hypoglycemia, hypoxemia and ischemia, as well as pharmaceutical formulations containing this pharmaceutically active agent.

Uridine is a well known nucleoside of uracil (D-ribosyluracil) of formula $C_9 H_{12} N_2 O_6$.

### 2. Description of the Prior Art

Uridine is already used in association with other pharmaceutical agents for other therapeutic indications. It has in fact been known for many years that pyrimidinic endogene nucleosides may play an important role in various physiological processes and may therefore be used to treat various pathological conditions.

For example, Geiger and co-workers (J. Neurochem. 1, 93 (1956)) have shown that cytidine and uridine can increase the survival time of isolated and perfused 'in situ' cat brain.

Elrick and co-workers (Metabolism 11, 46 (1962)) observed that cytidine and uridine used together restore normal metabolic conditions of glucose utilization in extra-hepatic tissues of patients suffering from hepatic cirrhosis. According to Cook and co-workers (Circ. Res. 6, 735 (1958)), uridine and cytidine exert a positive inotropic effect, improving ventricular efficiency. These discoveries led in Italy to the development of various drugs using uridine and/or cytidine, and their derivatives, in association with other substances, to treat hepatic dysfunctions and vascular cerebral pathologies.

More recent studies have shown that uridine may antagonize the epileptic discharges of metrazol and penicillin (Roberts: Brain Res. 55, 291 (1973)), and may be one of the physiological sleep inducers (Komoda and co-workers, Biomed Res. 4, 223 (1983)).

It has now surprisingly been discovered that uridine itself, administered to rats, is able to maintain neuron activity in cerebral distress models, and therefore may be useful in preventing the damage arising from cerebral aging and from other pathological conditions (for example hypoxemia, ischemia, hypoglycemia) which compromise neuron functionality.

In particular, computerized morphometry has shown that cholecystoquinin, a neuropeptide acting as a central nervous system transmitter which seems to be involved in neuroendocrinology and appetite control, is severely affected in conditions of severe insulin-induced hypoglycemia. However, if the animal is treated with uridine as well, it remains at normal levels. Furthermore, radioimmunological testing has shown that somatostatin, a neuropeptide with recognized modulatory activity toward many other neurohormones, is greatly reduced in the cerebral cortex and in the hippocampus following insulin-induced hypoglycemic coma, while it remains at normal levels if the animal is treated with uridine as well. It should be noted that somatostatin seems to be greatly reduced in the brains of elderly persons, especially in those with Alzheimer-type conditions.

Uridine's effect is not caused by variations in pH, $pO_2$ or $pCO_2$; nor is it related to increased blood sugar levels. Rather, it seems to derive from improved utilization of the

4

0178267

energy substrates by the brain, leading to maintenance of neuron functional activity even when blood circulation is low.

## Summary of the invention

Thus the object of this invention is a pharmaceutically active agent comprising uridine, useful in cerebral pathologies in elderly patients, under conditions of cerebral hypoglycemia and ischemia.

A further object of the invention is formed by pharmaceutical formulations comprising uridine as the pharmaceutically active agent and a pharmaceutically compatible carrier.

## Pharmacological tests

The effect of the pharmaceutically active agent according to the invention was evaluated on the basis of studies in rats.

Healthy Sprague Dawley rats weighing 250-320g were used, and were held under standard lighting conditions (lights on between 6:00 and 20:00). Fixed electrodes were implanted in the animals to record the EEG (electroencephalograms). After one week, the rats were kept fasting for 24 hours and then treated with insulin (40 i.u./Kg). One hour later preanesthesia was induced with halothane, followed by anesthesia with alpha-chloralose (100 mg/Kg). As soon as signs of isoelectric activity appeared in the EEG, they were injected venally over a 5 minute period with either 1 mL of physiological solution or 1 mL of physiological solution + uridine (15 mg/Kg).

## Test n. 1: Blood parameters

The blood of the animals treated as described above was tested to measure parameters such as pH, $pO_2$, $pCO_2$ and plasma glucose.

## Results:

No variation was observed in the pH, $pO_2$ and $pCO_2$ values for the animals treated with insulin and insulin + uridine, with respect to the non-treated control animals. Plasma glucose dropped from 4.9 millimoles/liter in the control animals to 0.9 millimoles/liter in the insulin-treated animals. Treatment with insulin + uridine gave the same results as treatment with insulin alone. Therefore, uridine may be assumed to have no effect on the insulin-induced glycemia curve, at least under these experimental conditions.

## Test n. 2: Effect on cholecystoquinin

After 40 minutes of isolectric activity in the EEG, the rats treated as described above were killed and their brains were removed, sectioned and treated with cholecystoquinine-specific antiserum. The area of the brain considered most carefuly was the hippocampus, where the neurons which utilize the above peptide as transmittor are more numerous and where the damage caused by prolonged hypoglycemia is most evident.

The 30 micron brain sections were incubated with antiserum, washed with phosphate buffer and treated with Sternberg's peroxidase-antiperoxidase system (Histochemistry 58, 167 (1979)). They were then transferred to glass slide frames and analyzed using a semiautomatic images analyzer hooked up to a computer.

The test was run using untreated animals (group 1), animals treated with insulin only (group 2), animals treated with uridine only (group 3) and animals treated with insulin + uridine (group 4). Each group was made up of 5 animals.

Results:

After 40 minutes of isoelectric activity on the EEG, a significant decrease in the number of neurons reacting to the cholecystoquinin antiserum was noted (only 40% of the neurons still reacted, with respect to the controls). In the animals treated with uridine, on the other hand, the number of neurons with cholecystoquinine remained practically the same. This shows that uridine can block the devastating effect on neurons of deep and prolonged hypoglycemia and can allow neuronic activity to continue even under the most unfavorable conditions.

Test n. 3: Effect on somatostatin

After 40 minutes of isoelectric activity on the EEG, the rats treated as described above were rapidly decapitated. The dorsal formation of the hippocampus (approximately 19mg) and the frontoparietal motor cortex (approximately 83mg) from each hemisphere were sectioned and frozen at $-70^{\circ}C$ until the radioimmunoassay was effected. The experiment involved the following groups of 7 rats each: controls, those treated with uridine, those treated with insulin, and those treated with insulin + uridine. The radioimmunoassay was carried out using the most common method, with somatostatin specific antibodies.

Results:

'The insulin-induced hypoglycemic coma leads to the disappearance of approximately 50% of the somatostatin in both the frontoparietal motor cortex and the hippocampus. On the other hand, treatment with uridine brought the somatostatin level to normal in the hippocampus, and even increased it with respect to basal values in the motor cortex. Since cerebral somatostatin decreases in cases of neuronic pathologies, uridine could antagonize cerebral degradation.

## Therapeutic indications

Uridine may be used to block the decrease in neuron activity which occurs as the brain ages, and in any case in which the neurons are deprived of the normal level of energy materials (hypoxemia, ischemia, hypoglycemia).

The pharmaceutically active agent according to this invention may be used clinically in pharmaceutical compositions for oral administrations, in the form of lozenges, pills, powders, capsules, drops, syrups and the like, together with pharmaceutically compatible excipients. The active pharmaceutical agent may be administered parenterally in the form of injectable solutes together with pharmaceutically acceptable carriers.

The preferred oral dose is 1-5g/day of the active pharmaceutical agent.

CLAIMS

1. Use of uridine for the manufacture of a medication for counteracting decay in neuron functional activity in brain pathologies.

2. Use of uridine according to claim 1 for the manufacture of a medication for the treatment of cerebral senility.

3. Use of uridine according to claim 1 for the manufacture of a medication for the treatment of cerebral hypoglycemia.

4. Use of uridine according to claim 1 for the manufacture of a medication for the treatment of cerebral hypoxemia.

5. Use of uridine according to claim 1 for the manufacture of a medication for the treatment of cerebral ischemia.

6. A pharmaceutical composition comprising an amount of uridine therapeutically effective in reducing deficits in neuron functional activity and a pharmaceutically compatible carrier.